## Europäisches Patentamt

## European Patent Office

## Office européen des brevets

(11) Veröffentlichungsnummer: **0 018 435**
**B1**

(12) **EUROPÄISCHE PATENTSCHRIFT**

(19)

(45) Veröffentlichungstag der Patentschrift:
**07.03.84**

(21) Anmeldenummer: **79103987.8**

(22) Anmeldetag: **16.10.79**

(51) Int. Cl.³: **G 01 N 33/48,** G 01 N 33/80,
G 01 N 33/54

(54) **Vorrichtung zur Durchführung von Mikroanalysen.**

(43) Veröffentlichungstag der Anmeldung:
**12.11.80 Patentblatt 80/23**

(45) Bekanntmachung des Hinweises auf die Patenterteilung:
**07.03.84 Patentblatt 84/10**

(84) Benannte Vertragsstaaten:
**AT BE CH DE FR GB IT NL SE**

(56) Entgegenhaltungen:
**AT - B - 170 898
DD - A - 96 784
DE - A - 2 527 770
US - A - 3 783 105**

(73) Patentinhaber: **Stöcker, Winfried, Dr. med.,
Krummesserweg 3, D-2419 Rondeshagen bei Lübeck
(DE)**

(72) Erfinder: **Stöcker, Winfried, Dr. med., Krummesserweg 3,
D-2419 Rondeshagen bei Lübeck (DE)**

(74) Vertreter: **UEXKÜLL & STOLBERG Patentanwälte,
Beselerstrasse 4, D-2000 Hamburg 52 (DE)**

## Vorrichtung zur Durchführung von Mikroanalysen

Die Anmeldung betrifft eine Vorrichtung zur Durchführung von klinisch-chemischen und immunologischen Untersuchungen.

a) Stand der Technik

Die Zahl klinisch-chemischer und immunologischer Untersuchungen nimmt ständig zu. Die Analysenverfahren werden daher rationalisiert und die Analysenansätze immer weiter verkleinert. Dadurch spart man Arbeitszeit, Platz, Material und Reagenzien. Eine Möglichkeit der Rationalisierung besteht darin, mehrere Proben gleichzeitig nebeneinander zu untersuchen und nicht nacheinander.

Für diesen Zweck hat sich das Vorgehen bewährt, mehrere Reaktionsgefässe in einer Platte zu integrieren wie bei dem von F. Mandula (AT-B-170 898) beschriebenen «Laboratoriumsgerät»: Die «Sekretentnahmeplatte» und die «Untersuchungsplatte» dieses «Laboratoriumsgerätes» enthalten jeweils mehrere nach demselben Muster angeordnete, in der Regel hohle «Sekretentnahmestellen» und «Sekretaufnahmestellen». Probenmaterial aus jeder einzelnen «Sekretentnahmestelle» der «Sekretentnahmeplatte» wird in die zugeordnete «Sekretaufnahmestelle» der «Untersuchungsplatte» übertragen und dort mit Reagenzien vermischt. Eine oder mehrere «Untersuchungsplatten» können in einen Rahmen gelegt werden, der zum Mischen der Proben mit den Reagenzien geschüttelt wird.

Die Proben werden bei diesem Verfahren einzeln nacheinander mit den Reagenzien zusammengebracht. Die Analysen der zuerst übertragenen Proben jeder Platte dauern daher länger als die Analysen der letzten Proben. Dadurch können Fehler entstehen, besonders wenn in einer Platte sehr viele Reaktionsgefässe vereinigt sind.

Auch bei der Technik nach R. H. Moyer und anderen (US-A-3 783 105) werden mehrere Analysen nebeneinander auf Platten durchgeführt. Drei Platten mit jeweils mehreren gleichartig angeordneten Bohrungen liegen übereinander, im Bereich der Bohrungen sind einige Scheibchen eingeklemmt oder anders befestigt. Die Scheibchen sind zu Säulen übereinandergelegt, zum Teil sind sie porös und mit Reagenzien imprägniert. Auf jede Bohrung der oberen Platte wird eine Probe getropft. Bestandteile der Probe diffundieren nach unten in die Säulen und reagieren mit den in den Scheibchen enthaltenen Reagenzien. Das Reaktionsprodukt wird durch einen transparenten Film, der die Säulen nach unten abschliesst, betrachtet oder im Auflicht photometriert.

Die Herstellung der imprägnierten Scheibchen, das Zusammensetzen der Platten und das Abdichten der Säulen sind aufwendig und schwierig, alle Reagenzien müssen gefriergetrocknet werden, jedes unterschiedliche Testverfahren verlangt eigene Testplatten. Darüber hinaus ist eine Photometrie im Durchlicht nicht möglich. In Abhängigkeit von der Reihenfolge der Proben bei der Analyse entstehen unterschiedlich lange Analysezeiten.

Demgegenüber werden bei dem von K. Thielmann und anderen angegebenen Verfahren (DD-A-96 784) viele Reaktionen gleichzeitig gestartet. Dazu werden zwei Platten mit jeweils mehreren Hohlräumen versehen. In die Hohlräume der einen Platte werden Proben gefüllt, in die Hohlräume der anderen Platte Reagenzien. Dann werden beide Platten so aufeinandergelegt, dass sich die einzelnen Hohlräume beider Platten gegenüberliegen und gemeinsame Reaktionsräume bilden. Die Platten werden zusammengepresst und dann alle Proben gleichzeitig durch Vibration mit den Reagenzien vermischt. Zur Auswertung müssen die Platten wieder voneinander getrennt und die Reaktionsprodukte auf ein Trägermaterial transferiert werden. Das ist umständlich und erlaubt auch keine kinetischen Messungen.

Bei den bisher genannten Verfahren sind die in Platten zusammengefassten Reaktionsgefässe hohl, das Reaktionsgemisch wird in jedem einzelnen Reaktionsgefäss nach mindestens fünf Richtungen hin von der Gefässwand umgeben: nach vier Seiten und nach unten. Durch einen Träger, der das Reaktionsgemisch nach nur einer oder zwei Richtungen hin umgibt, kann der Platz- und Materialbedarf weiter reduziert werden.

Bei der Blutgruppenbestimmung kann man beispielsweise eine oder mehrere Reaktionen nebeneinander auf einem Objektträger ablaufen lassen oder auch bei Immunfluoreszenzuntersuchungen an Gefrierschnitten (Beschreibung z.B. bei Seelig, H. P. und anderen, Lab. med. 2, 1978, 133–139).

Die Oberfläche des Objektträgers kann in Reaktionsfelder unterteilt werden, die voneinander durch eine inerte, wasserabstossende Schicht getrennt sind. Verschiedene, nebeneinanderliegende Analysenansätze vermischen sich dann nicht so leicht. Die z.B. bei O'Neill, P. und Johnson, G. D.; Ann. N. Y. Acad. Sci. 177, 1971, 446–452, beschriebene Beschichtung mit Polytetrafluoroethylen hält jede Probe auf ihrem Reaktionsfeld fest und verhindert, dass man beim Betrachten des Analysenergebnisses durch Lichtreflexionen an der Umgebung der Reaktionsflächen gestört wird, beispielsweise bei einer Blutgruppenuntersuchung. Ein Aufrauhen der Glasoberfläche in der Umgebung der Reaktionsfelder führt ebenfalls zu einer Probentrennung (Berndt, H. und Timmermann, E.-O.; DE-A1-2 527 770), ist aber weniger wirksam als die Beschichtung mit Polytetrafluoroethylen.

Die Tropfen trocknen auf dem Objektträger leicht an, auch wenn er sich in einer feuchten Kammer befindet. Dadurch kann die Analyse verfälscht werden (Anstieg der Konzentration gelöster Substanzen) oder verdorben. Deshalb wendet man häufig eine Technik an, wie sie Tung, K. S. K. empfiehlt (J. Immunol. Methods 18, 1977, 391–392): Ein weiterer, unbeschichteter Objektträger wird mit Abstand so darübergelegt, dass er die Tropfen berührt. Dabei laufen allerdings häufig die verschiedenen Proben zusammen und vermischen sich, besonders dann, wenn sich am oberen Objektträger ein Kondenswasserfilm gebildet hat, wenn die beiden Objektträger gegeneinander verrutschen oder wenn die Tropfen nicht genau plaziert sind. Die Tropfen müssen genügend weit voneinander entfernt sein, damit sie nicht zusammenlaufen. Das steht einer Verkleinerung der Analysenanordnung entgegen, die Methode kann in dieser Form auch nicht leicht automatisiert werden.

A. Horn und andere beschreiben einen Probenträger aus zwei Platten mit Reaktionsfeldern (DD-PS 107 783). Die Reaktionsfelder sind von ihrer Umgebung durch Nuten, Vorsprünge oder Vertiefungen abgesetzt. Auf die Reaktionsfelder der einen Platte werden Proben, auf die anderen Platte Reagenzien getropft. Dann legt man die Platten mit einem bestimmten Abstand so übereinander, dass sich die Reaktionsfelder gegenüberliegen und die einander zugeordneten Proben- und Reagenzientropfen miteinander verschmelzen. Dadurch werden die Reaktionen gleichzeitig gestartet, gleichgültig wie viele Reaktiosfelder auf den Platten vorhanden und betropft sind. Die Reaktionsgemische bilden Flüssigkeitssäulen aus, die von je zwei sich gegenüberliegenden Reaktionsfeldern durch Adhäsionskräfte festgehalten werden. Zum Mischen wird der Plattenabstand periodisch verändert und dadurch eine Konvektion in der Flüssigkeit hervorgerufen. Die Platten sind im Bereich der Reaktionsfelder strahlendurchlässig und ermöglichen eine Photometrie senkrecht zur Plattenebene.

Die einzelnen Analysenansätze werden bei diesem Verfahren durch eine geeignete räumliche Strukturierung der Plattenoberflächen auseinandergehalten. Daraus ergibt sich bei Agglutinationsreaktionen eine Fehlerquelle: Gelegentlich führt eine Blutgruppenanalyse zu einem falschen Resultat, weil Agglutinate (Komplexe aus roten Blutkörperchen mit den korrespondierenden Antikörpern) nach den Seiten hin aus dem Bereich der Reaktionsfelder verdrängt werden und beispielsweise in der Nut um die Reaktionsfelder liegenbleiben, wo sie der Sicht entzogen sind.

Ausserdem verdunstet Wasser aus den Analysenansätzen und schlägt sich an beiden Plattenoberflächen ausserhalb der Reaktionsfelder nieder, und zwar um so mehr, je länger die Reaktionen dauern und je höher die Reaktionstemperatur und die relative Luftfeuchtigkeit sind. Dabei verändern sich die Konzentrationen in den Analysenansätzen. Der Kondensationsfilm schlägt Brücken zwischen den Reaktionsfeldern, und die Tropfen verlaufen manchmal und vermischen sich untereinander.

Die Oberfläche der Platten weist eine komplizierte räumliche Strukturierung auf. Die Herstellung ist daher aufwendig und erfordert teure Werkzeuge.

b) Prinzip der Erfindung und Abgrenzung zum Stand der Technik

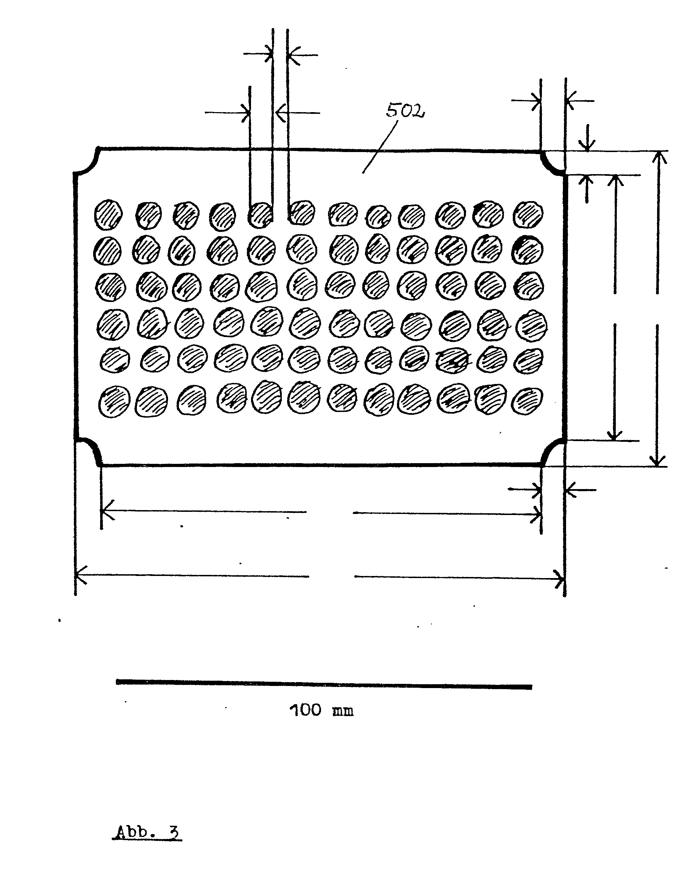

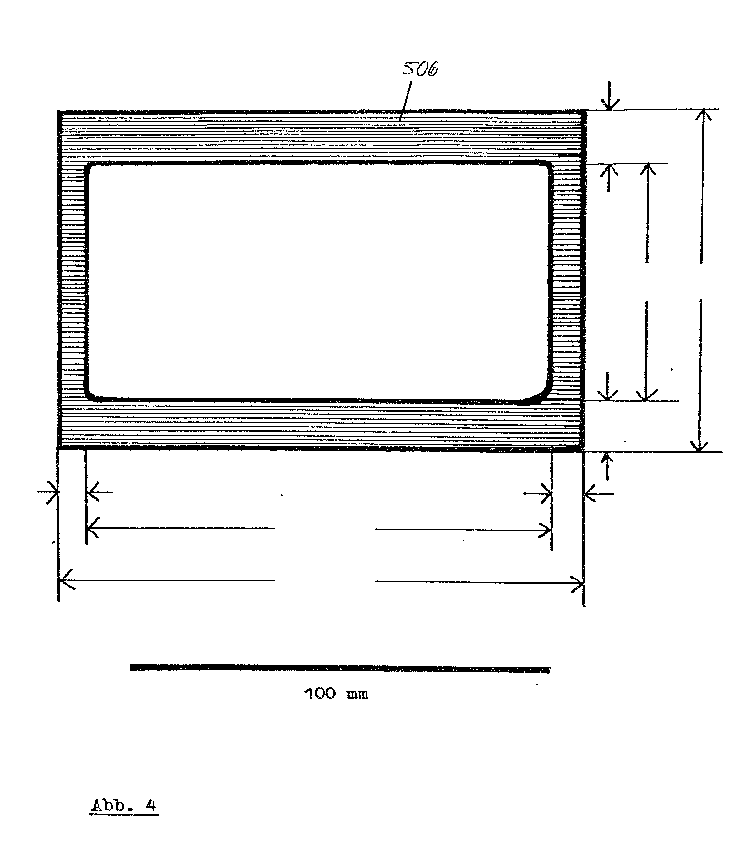

Zwei gleichgrosse Glas- oder Kunststoffplatten werden auf je einer Fläche mit einer wasserabweisenden Substanz so beschichtet, dass eine bestimmte Anzahl Kreisflächen freibleibt (Beispiel für eine Anordnung der Kreisflächen: Abb. 3).

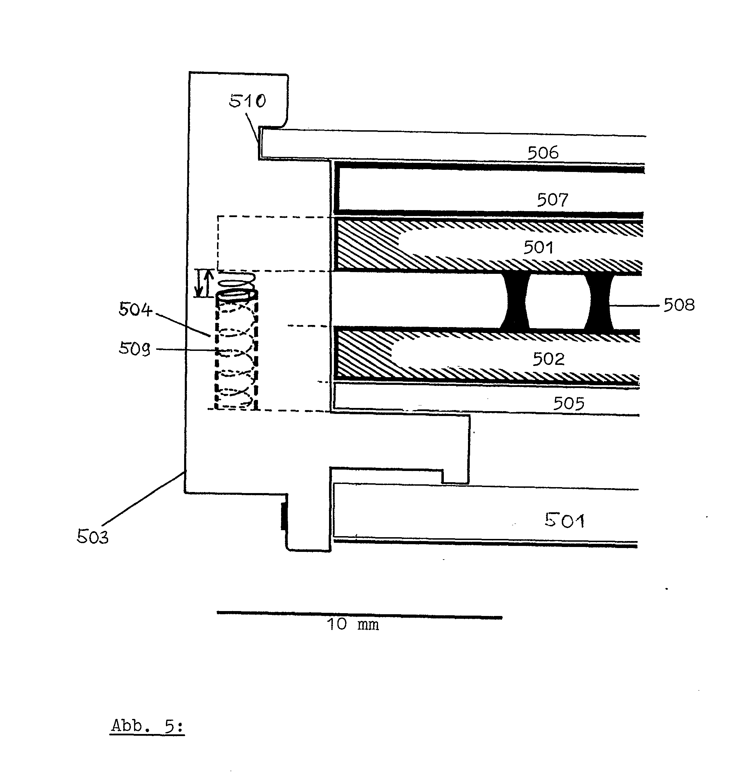

Gemäss Abb. 5 wird eine der beiden Platten mit der Schichtseite nach oben horizontal in ein Grundgestell 503 gelegt (Unterplatte 502). Auf jede Kreisfläche der Unterplatte wird ein definiertes Volumen einer wässrigen Lösung gebracht.

Die andere Platte (Oberplatte 501) wird, mit der Schichtseite nach unten, darübergelegt. Die Kreisflächen beider Platten liegen deckungsgleich übereinander, beide berühren die Tropfen. Das Grundgestell ist so konstruiert, dass Oberplatte und Unterplatte nicht horizontal gegeneinander verrutschen können und dass beide Platten einen definierten Mindestabstand voneinander einhalten. Die Tropfen 508 zwischen den Kreisflächen werden mit starken Adhäsionskräften festgehalten, weil die unbeschichteten Flächen der Unterplatte und der Oberplatte Wasser mit einer wesentlich grösseren Adhäsionskraft anziehen, als die wasserabweisend beschichteten Flächen es tun. Die Tropfen werden auch dann festgehalten, aber etwas schwächer, weil nur von der Unterplatte, wenn die ganze Unterfläche der Oberplatte beschichtet ist oder wenn die Oberplatte aus einem wasserabstossenden Kunststoff besteht.

Die Platten werden rhythmisch parallel aufeinander zu und voneinander weg bewegt, ohne dass die Tropfen zwischen den Platten abreissen und ohne dass die Tropfen so breit gequetscht werden, dass sie sich untereinander vermischten. Durch diese Verformung der Tropfen werden Substanzen, die sich in den Tropfen befinden, gemischt. Die Plattenbewegung wird durch einen Magneten und durch Federn bewirkt, es wäre aber auch eine andere Vorrichtung denkbar, z.B. ein Nockenwellen-Federn-System.

Die Dimensionen der Kreisflächen, des Plattenabstandes und der Tropfendurchmesser können variiert werden, es wurden Versuche einer Miniaturisierung bis herab zu 25 Reaktionsfeldern pro Quadratzentimeter erfolgreich durchgeführt.

c) Aufbau und Funktion des Instruments

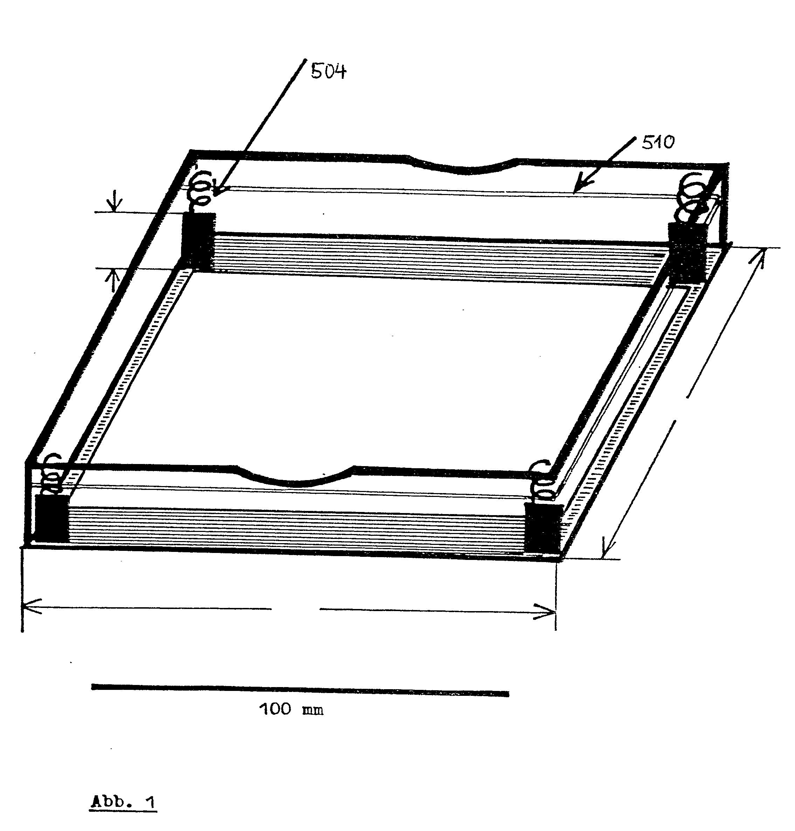

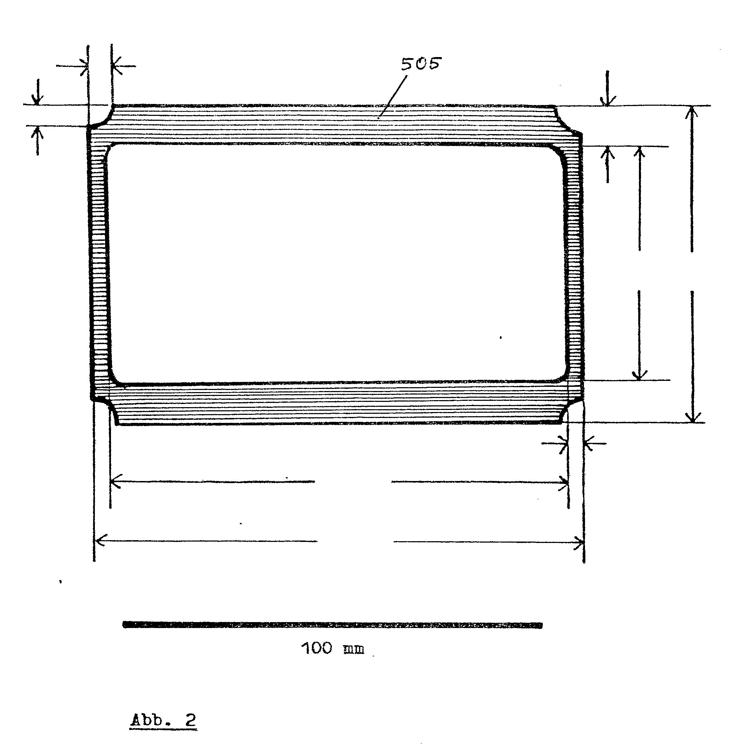

Das Grundgestell 503, (Abb. 1, Abb. 5) besteht aus einem stabilen Aussenrahmen, in den innen rundum ein leistenartiger Boden integriert ist. Auf den Boden werden übereinandergelegt: 1 Distanzrahmen 505, 1 Unterplatte 502, 1 Oberplatte 501, 1 Eisenplatte 507. Darüber wird ein Sperrahmen 506 geschoben, der in einer Fuge 510 im Aussenrahmen des Grundgestells gleitet. An den vier Ecken trägt der Boden je einen Abstandhalter 504, der genau 4 mm nach oben reicht. Jeder Abstandhalter enthält eine senkrechte zylinderförmige Aussparung, die eine Teleskopfeder 509 aufnimmt. Diese Feder lässt sich so weit eindrücken, dass sie den Abstandhalter nicht mehr überragt, im entspannten Zustand steht sie um 3,0 bis 3,5 mm über. Der Boden ist innen, im Bereich der Kreisflächen der Ober- und Unterplatte, offen. Er hat die gleichen Ausmasse wie der Distanzrahmen, abgesehen davon, dass der Distanzrahmen an seinen Ecken Aussparungen für die Abstandhalter hat, die im Boden verankert sind, vgl. Abb. 1 und Abb. 2. Distanzrahmen gibt es in unterschiedlicher Stärke. Man kann mit ihnen den Minimalabstand zwischen Ober-und Unterplatte festlegen: Die Oberplatte wird von den vier 4 mm hohen Abstandhaltern zurückgehalten. Die Unterplatte liegt auf dem Distanzrahmen und wird, je nach desses Stärke, unterschiedlich nahe an diese 4-mm-Grenze gehoben. Bei Verwendung grosser Tropfen legt man die Unterplatte niedriger als bei kleinen Tropfen. Die Unterplatte ist in Abb. 3 dargestellt. Die Oberplatte ist wie ein Spiegelbild der Unterplatte beschichtet, damit jeder Tropfen 508 zwischen zwei unbeschichtete Kreisflächen kommt. Die Oberplatte hat an den Ecken keine Aussparungen und liegt daher auf den vier Federn. Presst man sie stark nach unten, dann drückt sie die Federn ganz in die Abstandhalter hinein und stösst an diese an. Dabei werden die Tropfen breitgedrückt. Lässt man die Oberplatte wieder los, dann wird sie durch die Federn nach oben gehoben. Dabei werden die Tropfen verformt und in die Höhe gezogen. Nimmt man die Oberplatte ganz ab, dann teilen sich die Tropfen, der grössere Teil bleibt auf den Kreisflächen der Unterplatte liegen, der kleinere Teil hängt an den Kreisflächen der Oberplatte.

Die Tropfen werden durch eine rhythmische Verformung effektiv gemischt. Dazu wird eine Eisenplatte auf die Oberplatte gelegt, die von einem unter dem Grundgestell befindlichen Elektromagneten in bestimmten Abständen ruckweise angezogen wird. Immer wenn der Magnet sich ausschaltet, wird die Oberplatte mit der darüberliegenden Eisenplatte von den Federn abgestossen. Es ist zweckmässig, einen Elektromagneten einzusetzen, dessen Impulsfrequenz, Impulshärte und Kraftfluss variierbar sind. Die Eisenplatte ist mit Löchern versehen, die sich mit den Kreisflächen der Ober- und Unterplatte decken.

Damit sich die Oberplatte nicht zu weit von der Unterplatte entfernen kann, wird über die Eisenplatte ein Sperrahmen in die dafür vorgesehene Fuge des Grundgestells geschoben. Dieser lässt sich am Grundgestell durch zwei Stifte arretieren. Sollen am Ende der Analyse die Tropfen flachgedrückt werden, dann muss man unter den Sperrahmen noch einen Ableserahmen legen. Dadurch breiten sich z.B. bei der Blutgruppenbestimmung die Zellklümpchen aus und stellen sich viel deutlicher dar.

Das umgedrehte Grundgestell kann eine mikroskopisch zu untersuchende Oberplatte und ein darübergelegtes gleichgrosses Deckglas aufnehmen. Beide können gegeneinander nicht verrutschen, weil sie nach den Seiten hin durch den Aussenrahmen des Grundgestelles begrenzt werden.

Das Grundgestell ist stapelfähig (vgl. Abb. 5). Zur leichteren Handhabung der Platten sind an seinen Längsseiten Greifrinnen angebracht, die nicht ganz bis an die Schicht der Tropfen hinunterreichen, damit diese nicht trocknen können.

Oberplatten und Unterplatten aus Glas werden wie folgt beschichtet: Sie werden zuerst sorgfältig gereinigt und entfettet. Mit einem Gummistempel wird eine Schicht warmen, flüssigen Fetts auf die Felder gebracht, die frei von der wasserabweichenden Schicht bleiben sollen. Darüber wird eine Lösung gesprüht, die z.B. Polytetrafluoroäthylen enthält. Danach wird das Fett heiss abgewaschen, und die Platten werden getrocknet und poliert. Da man nicht leicht feststellen kann, auf welcher Seite sich die Schicht befindet, und damit man die Proben sicher identifizieren kann, werden die Platten an einer bestimmten Stelle der Ränder zur beschichteten Fläche hin mit einem Farblack markiert.

d) Beispiele
Beispiel 1:
Einsatz der Erfindung bei der Blutgruppenbestimmung
Prinzip:
Rote Blutkörperchen einer bestimmten Blutgruppe werden durch Antikörper gegen diese Blutgruppe verklumpt. Für eine normale Blutgruppenbestimmung werden in der Regel etwa 14 Einzeltests durchgeführt (ABO-System und aufgeschlüsseltes Rhesussystem).

Bisherige Methode (Beispiel einer einfachen Methode ohne aufwendige Geräte):
Je Einzeltest werden in ein Reagenzglas oder auf einen Objektträger zu 10 bis 50 µl Aufschwemmung roter Blutkörperchen 10 bis 50 µl Antiserum gegeben. Die Röhrchen werden geschüttelt, die Tropfen auf den Objektträgern mit einem Stäbchen umgerührt (für jeden Tropfen ein frisches Stäbchen!). Ein Teil der Ansätze wird in einem Brutschrank zur Reaktion gebracht. Die Proben werden mit dem blossen Auge auf Verklumpung untersucht.

Nachteile dieser Methode: hoher Proben- und Reagenzienverbrauch, material- und zeitaufwendiges Mischen. Die Tropfen trocknen innerhalb einiger Viertelstunden an und sind dann nicht mehr sicher zu beurteilen.

Erfindungsgemässe Bestimmung der Blutgruppe:
In das Grundgestell wird ein Distanzrahmen von 1,9 mm Breite gelegt und darauf eine Unterplatte, wie in Abb. 3. Um zu verhindern, dass die Tropfen beim Auftragen antrocknen, wird vorübergehend die Eisenplatte, die an den Auftragsstellen durchlöchert ist, direkt auf die Unterplatte gelegt. In jedes Reaktionsfeld werden 2 µl Blutkörperchen und 2 µl Antikörperlösung pipettiert. Dazu können teilweise handelsübliche Mehrkanalpipetten verwendet werden. Nach dem Betropfen wird die Eisenplatte abgenommen, die Oberplatte wird aufgelegt, darüber kommen die Eisenplatte und der Sperrahmen. Das Ganze wird auf den Elektromagneten gestellt, der den Abstand zwischen Ober- und Unterplatte rhythmisch zwischen 0,1 und 1,1 mm wechseln lässt und dadurch die Reaktionspartner innerhalb der einzelnen Tropfen vermischt. Ein Teil der Reaktionen erfolgt in einem Brutschrank bei 37 °C. Nach Ablauf der Reaktionszeit wird der Elektromagnet ausgeschaltet, und unter den Sperrahmen kommt eine 0,9 mm breiter Distanzrahmen. Dadurch werden die Tropfen breitgedrückt, und die verklumpten Blutkörperchen stellen sich deutlicher dar.

Vorteile dieser Methode:

Niedriger Proben- und Reagenzienverbrauch, das Mischen erfolgt vollautomatisch. Die Tropfen sind nach 24 Stunden noch nicht sichtbar eingetrocknet. Die breitgedrückten Verklumpungen sind, gegen eine diffuse Lichtquelle gehalten, brillant dargestellt, viel deutlicher als mit der bisherigen Methode. Die Ergebnisse lassen sich gut im Durchlicht photographieren oder bei Vorträgen mit Durchlichtprojektoren demonstrieren.

Beispiel 2
Einsatz der Erfindung in einem Enzym-Immunoassay
Prinzip und bisherige Methode:

Die verschiedenen Arten der Enzym-Immunoassays werden u. a. von Eggstein, M., Liebich, H. M., Stein, W. (Diagnostik 10, 1977, 74*–78*) beschrieben. Als eine von mehreren Möglichkeiten wird im folgenden der Sandwich-Enzym-Immunoassay oder Festphasen-Enzym-Immunoessay dargestellt: Mit einer Substanz, deren Menge im Test bestimmt werden soll (Antigen), wurden vor dem Test Tiere aktiv immunisiert und aus dem Blut dieser Tiere Antikörper isoliert. Ein Teil dieser Antikörper wurde an den Boden von Teströhrchen chemisch oder pysikalisch gebunden (vgl. dazu Offenlegungsschrift 28 16 830, Deutsches Patentamt, G 01 N 31/16). Der andere Teil der Antikörper wurde durch ein Enzym markiert.

Im Test wird das nachzuweisende Antigen in gelöster Form in das Röhrchen gegeben und bleibt an der Schicht der im Überschuss vorhandenen Antikörper hängen. Der Überstand wird am Ende der Inkubation abgekippt und das Röhrcheninnere mehrmals gewaschen. Enzymmarkierter Antikörper wird zugesetzt und bindet sich überall dort an die Wand des Röhrchens, wo sich vorher Antigen fixiert hat. Der Überstand wird am Ende der zweiten Inkubation wieder abgekippt und das Röhrcheninnere mehrmals gewaschen. Zum Nachweis des an das Röhrcheninnere gebundenen Enzyms wird ein Reagenzgemisch in das Röhrchen gegeben, dessen Farbzusammensetzung sich ändert, wenn das entsprechende Enzym anwesend ist. Die Farbänderung wird mit einem Photometer gemessen und daraus die eingesetzte Antigenmenge berechnet. Eine grosse Farbänderung zeigt eine hohe Antigenmenge an.

Nachteile der Methode:

Die Proben werden während der Inkubationen nicht ausreichend durchgemischt, dadurch wird die Versuchszeit ausgedehnt. Das Mischen ist besonders ineffektiv in Mikrotiterplatten, weil sich die Flüssigkeit in den engen Vertiefungen durch einfaches Schütteln nur wenig bewegen lässt.

Das erforderliche mehrmalige Waschen ist umständlich, weil jedes einzelne Gefäss mehrmals gefüllt und entleert werden muss. Dieses Verfahren lässt sich nicht viel weiter miniaturisieren, es verbraucht viel Probe und markierten Antikörper.

Die beschriebenen Nachteile werden mit einer Methode umgangen, bei der magnetische Eisenkugeln als Träger der Antikörper verwendet werden (Smith, K.O., Gehle, W.D.; J. Infect. Dis. 136, 1977, S. 329–336). Diese Magnetkugeln sind allerdings etwas umständlich zu handhaben. Es werden auch noch viel Probe und Antikörper verbraucht. Das liegt daran, dass die beschichteten Flächen immer bedeckt sein müssen und dass das eingesetzte Volumen wesentlich grösser sein muss als das Volumen, das während der Inkubationen verdunstet.

Erfindungsgemässe Durchführung des Festphasen-Enzym-Immunoassay:

In das Grundgestell wird ein Distanzrahmen von 1,9 mm Stärke eingelegt und darauf eine Unterplatte (wie in Abb. 5). Um zu verhindern, dass die Tropfen beim Auftragen antrocknen, wird vorübergehend die durchlöcherte Eisenplatte direkt auf die Unterplatte gelegt. In jedes Reaktionsfeld werden 5 µl Probe vorgelegt. Die Eisenplatte wird wieder abgenommen und die Oberplatte eingelegt. Die Reaktionsfelder der Oberplatte zwischen der wasserabstossenden Schicht wurden zuvor mit Antikörpern beschichtet, ähnlich wie die Teströhrchen bei der bisherigen Methode. Darüber kommen Eisenplatte und Sperrahmen, und der Elektromagnet wird eingeschaltet. Die Tropfen werden dadurch rhythmisch so verformt, dass ihre Höhe zwischen 0,1 mm und 1,1 mm wechselt. Nach Ablauf der Inkubationszeit wird die Oberplatte abgenommen und kurz mit Waschlösung abgespritzt. Dann kommt sie in ein Waschgestell, das die Platte durch zwei seitliche Schlitze festhält, ähnlich wie ein Diapositivbehälter das Dia. Die Platten haben in den Schlitzen etwas Spiel, oben etwas mehr als unten. Das Waschgestell ist nach unten und oben offen und wird in einen Behälter mit Waschflüssigkeit gestellt. Der Behälter wird von einem Schüttler bewegt. Schüttelfrequenz und Spiegel der Waschflüssigkeit werden so eingestellt, dass die Oberkanten der Platten gleichzeitig entgegen der Bewegung des Schüttlers kippen. Dadurch wird eine gleichmässige, rhythmisch wechselnde Flüssigkeitsströmung über den Oberplatten erzeugt, wodurch die Waschzeiten im Vergleich zu einer ruhenden Methode erheblich verkürzt werden können.

Die Waschflüssigkeit kann mehrmals erneuert werden.

Nach dem Waschen werden die Felder der Oberplatte mit 5 µl enzymmarkiertem Antikörper inkubiert. Zuvor kann die auf den Feldern liegengebliebene Waschflüssigkeit entfernt werden, da sie die Antikörperlösung verdünnen könnte. Dazu wird die Oberplatte, mit der Beschichtung nach unten, auf ein feines Filterpapier gedrückt. Nach der Markierung wird erneut abgespült und gewaschen.

Danach wird der Distanzrahmen aus dem Grundgestell genommen und eine neue Unterplatte direkt auf den Boden des Grundgestells gelegt. Die Kreise der Unterplatte erhalten je 50 µl Farbreagens mit einer Mehrkanalpipette. Oberplatte, Eisenplatte und Sperrrahmen werden darübergelegt, und der Elektromagnet wird eingeschaltet. Der Abstand zwischen Ober- und Unterplatte wird zwischen 2 mm und 3 mm rhythmisch verändert.

Zur Photometrie wird der Elektromagnet ausgeschaltet, und das ganze System wird in ein Photometer gestellt, das für die Lichtmessung an Mikrotiterplatten geeignet ist, z.B. ein Gerät der Firma Flow Laboratories (5309 Mechenheim, Mühlgrabenstrasse 10, Deutschland): Titertek[®] Multiskan.

Um die Empfindlichkeit der Methode zu steigern, kann die Unterplatte bei der Enzymreaktion anstelle der Reaktionsfelder auch zylindrische Vertiefungen haben, die bis 5 mm unter den Grundgestellboden reichen.

Vorteile der Erfindung im Enzym-Immunoassay:

In jeder Phase des Tests werden die Reaktionslösungen effektiv gemischt, wodurch sich die Versuchszeiten verkürzen.

Beim Waschen zwischen den Inkubationen wird besonders viel Zeit eingespart, weil in einem Gestell gleichzeitig viele Platten gewaschen werden können, z.B. 20 oder 40 Platten, entsprechend 1440 oder 2880 einzeltests.

Es wird viel weniger an Proben und an enzymmarkiertem Antikörper gebraucht als bei allen früheren Methoden, besonders wenn die Methode noch weiter miniaturisiert wird: Die Oberplatte kann mit 1 mm grossen Reaktionsfeldern versehen werden, die einen Abstand von 1 mm voneinander haben (25 Enzym-Immunoassays auf 1 Ouadratzentimeter!). Zum Auftragen kann man Kleinvolumen-Mehrkanalpipetten verwenden, deren Spitzen den gleichen Abstand voneinander haben wie bei den zurzeit gängigen Multipipetten (9 mm). Man muss nur mehrmals und um jeweils 2 mm versetzt auftragen. Die Indikatorreaktion mit dem enzymmarkierten Antikörper kann in einem dünnen, zum Schluss über die Oberplatte gegossenen Gel erfolgen, das einen geeigneten Farbgeber enthält. Automatische Messung in einem Mikroskop mit eingebautem Photometer.

Beispiel 3
Einsatz der Erfindung in einem Radioimmunoassay

Einer der möglichen Radioimmunoassays ist der Festphasen-RIA (Sandwich-RIA). Er ist dem in Beispiel 2 beschriebenen Sandwich-Enzym-Immunoassay vergleichbar, anstelle mit Enzym werden Antikörper mit Radioisotopen markiert. Eine allgemeine Beschreibung des Festphasen-RIA findet sich in der Offenlegungsschrift 2 816 830, Deutsches Patentamt, G 01 N 31/16. Im Test wird die Enzym-Farbreaktion durch eine Radioaktivitätsmessung ersetzt. Wenn Gammastrahler verwendet werden, muss die Radioaktivität auf den Reaktionsflächen der Oberplatten einzeln im Zählgerät gemessen werden. Dazu empfiehlt es sich, Oberplatten aus Plastik zu verwenden und sie von oben her so vorzustanzen, dass sich die Reaktionsflächen leicht herausdrücken lassen, ggf. durch eine geeignete Mehrfachstempel-Konstruktion. Werden Beta-Strahler eingesetzt, dann kann die Oberplatte autoradiographiert werden. Anschliessend wird die Silberkorndichte der Autoradiogramme mit einem Mikrophotometer gemessen, wie z.B. angegeben von Hughes, H.C. et al.; Stain Technol. 52, 1977, 79–83.

Beispiel 4
Einsatz der Erfindung in einem Fluoro-Immunoassay

Dieser Test entspricht dem Enzym-Immunoassay und dem Radioimmunoassay, anstelle mit Enzymen oder mit Radioisotopen werden die Antikörper mit fluoreszierenden Substanzen markiert, z.B. mit Fluoresceinisothiocyanat. Am Ende der Tests wird die Fluoreszenzintensität der Reaktionfelder in der Oberplatte durch ein automatisches Fluoreszenzphotometer gemessen.

Beispiel 5
Einsatz der Erfindung in einem Immunfluoreszenztest an histologischen Gefrierschnitten. Herstellen einer Verdünnungsreihe

Mit dieser Methode können Antikörper gegen das körpereigene Gewebe nachgewiesen werden. Sie wurde von Coons erfunden (Coons, A.H.; Creech, H.J., Jones, R.N.; Proc. Soc. exp. Biol. N. Y. 47, 1941, S. 200 ff). Eine moderne Beschreibung findet sich bei Seelig, H.P., Geisen, H.P., Seelig, R.; Lab. med. 2, 1978, 133–139. Die Immunfluoreszenz lässt sich mit der vorliegenden Erfindung besonders rationell durchführen. Dazu werden die Reaktionsfelder der Oberplatte mit Gefrierschnitten oder mit Zellen beschichtet. Im indirekten Test werden diese Präparate zuerst mit der Probe und nach dem Waschen mit einer Lösung fluoresceinmarkierter Antikörper inkubiert (Einzelheiten siehe Beispiel 2).

Nach dem letzten Waschzyklus wird die Oberplatte in die vorgesehene Aussparung eines umgedrehten Grundgestells gelegt. Auf jedes Feld werden 5 µl Einbettungsmedium getropft, und ein Deckglas von der Grösse der Oberplatte, 0,1 mm dick, wird darübergelegt. Oberplatte und Deckglas werden vom Grundgestell so eingefasst, dass sie gegeneinander nicht verrutschen können (dadurch würden die Präparate zerstört). Falls nach unkorrektem Auftropfen überschüssiges Einbettmedium zwischen Objektplatte und Deckglas hervorquellen sollte, wird dieses vom Grundgestell zurückgehalten und tropft nicht auf das Mikroskop (was bisher immer wieder zu einem Versagen des Transports durch den Kreuztisch führte). Da eine Objektplatte mit z.B. 72 Feldern versehen ist, entfällt das häufige Wechseln der Präparate.

Bei Immunfluoreszenzuntersuchungen muss häufig die Konzentration der Autoantikörper in den Proben gemessen werden. Dazu werden Verdünnungsreihen angesetzt und festgestellt, bei welcher Verdünnung noch Fluoreszenz nachzuweisen ist. Bisher war es üblich, diese Verdünnungen in besonderen Gefässen herzustellen. Die hier beschriebene Erfindung bietet die Möglichkeit, die Proben direkt auf der Unterplatte zu verdünnen. Auf das erste Feld jeder Verdünnungsreihe kommen z.B. 20 µl Probe, auf die anderen Felder je 20 µl Verdünnungslösung. Mit einer 20-µl- Kolbenhubpipette werden weitere 20 µl Probe auf das zweite Feld gebracht und durch mehrmaliges Aufsaugen und Ausstossen mit der Verdünnungslösung vermischt. Von dieser 1:1-Verdünnung werden mit derselben Pipettenspitze 20 µl auf das nächste Feld übertragen und dort mit der Verdünnungslösung vermischt usw. Von den letzten 40 µl werden 20 µl verworfen. Die Titerschritte können durch Variation der vorgelegten und der transportierten Volumina beliebig bemessen werden.

Beispiel 6

Einsatz der Erfindung bei Substratbestimmungen in der klinischen Chemie

Die Erfindung eignet sich auch für Substratbestimmungen, wie Glucose oder Harnstoff, da sie die Möglichkeit zu einer gleichzeitigen Photometrie vieler Proben bietet, vgl. Enzym-Farbreaktion im Enzym-Immunoassay. Als besonders aussichtsreich erscheint die Möglichkeit, Enzyme fest an die Reaktionsfelder der Oberplatte zu binden, die bei Substratanalysen mitwirken (z.B. Werner, M. et al.; Clin. Chem. 25, 1979, 20–23). Eine solche Enzym-Reaktor-Oberplatte ist wiederverwendbar.

Weiterhin ist es möglich, eine Oberplatte mit den für eine Substratanalyse erforderlichen Reagenzien vorzubeschichten. Die Proben und eine Pufferlösung werden auf die Felder der Unterplatte gebracht und die Reaktion durch Übereinanderlegen der Platten und Einschalten des Elektromagneten in Gang gesetzt. Es kann nach Abschluss der Reaktion photometriert werden (Endpunktmessung) oder mehrmals während der Reaktion (kinetische Methode). Für die grosse Routine werden auf einer Oberplatte alle Felder mit dem gleichen Reagens versehen oder, für das Notfall-Screening im klinischen Labor, jedes Feld einer Oberplatte mit verschiedenen Reagenzien.

**Patentansprüche**

1. Vorrichtung zur Durchführung von Mikroanalysen einer oder mehrerer gelöster oder in Lösung zu bringender Proben bei immunologischen Untersuchungen, insbesondere Blutgruppenbestimmungen, Radioimmunoassays, Enzymimmunoassays, Fluoreszenzimmunoassays, und bei nichtimmunologischen allgemeinchemischen, biochemischen und klinisch-chemischen Untersuchungen, bestehend aus

zwei Platten (501, 502), die auf je einer Fläche mit Reaktionsfeldern versehen sind,

einem Gestell (Grundgestell 503), das die beiden Platten aufnimmt, sie parallel zueinander anordnet und sie daran hindert, seitlich gegeneinander zu verrutschen,

Mitteln (504, 505) zur Einstellung eines kleinsten Abstandes beider Platten voneinander,

Mitteln (506, 507) zur Einstellung eines grössten Abstandes beider Platten voneinander und

Mitteln zur Veränderung des Abstandes beider Platten voneinander zwischen seinem grössten und seinem kleinsten Wert, dadurch gekennzeichnet, dass die Reaktionsfelder der aus Glas oder Kunststoff bestehenden Platten hydrophil ausgebildet und von einer wasserabstossenden Schicht umgeben sind, sich deckungsgleich gegenüberliegen und die Proben (508) von beiden Seiten festhalten.

2. Vorrichtung nach Anspruch 1 zur gleichzeitigen Mischung der Proben (508) zwischen den beiden Platten (501, 502), dadurch gekennzeichnet, dass die Mittel zur Veränderung des Abstandes beider Platten voneinander aus einer von einem Elektromagneten rhythmisch angezogenen Eisenplatte (507) und aus Druckfedern (509) zur Erzeugung eines Gegendrucks bestehen.

**Claims**

1. Apparatus for performing microanalyses of one or a plurality of dissolved or dissolvable specimens in immunological examinations, particularly blood group determinations, radio-immuno assays, enzyme-immuno assays, immunofluorescent assays, as well as in non-immunological general chemical, biochemical and clinical-chemical examinations, consisting of

two plates (501, 502) provided with reaction fields on each one surface,

a frame (base frame 503) receiving the two plates, arranging them parallelly to each other and preventing them to slide transversely relative to each other, means (504, 505) for adjusting a minimum distance between each plates,

means (506, 507) for adjusting a maximum distance between each plates and

means for varying the distance of each plates relative to each other between said maximum and said minimum distance, characterized in that

the reaction fields of the glass or plastic plates are made hydrophilic and are surrounded by a water-repellant coating, said reaction fields lying in registry relative to each other and holding the specimens (508) at both sides.

2. Apparatus according to claim 1 for simultaneously mixing the specimens (508) between the two plates (501, 502), characterized in that

the means for varying the distance between both plates comprises an iron plate (507) rhythmically attracted by an electromagnet and further comprises compression springs (509) for generating a counterpressure.

**Revendications**

1. Dispositif pour la conduite de microanalyses d'un ou plusieurs échantillons dissous ou à amener en solution pour des recherches immunologiques, des déterminations de groupes sanguins, des essais radioimmunologiques, des essais immunoenzymotiques, des essais en immunofluorescence et des recherches non immunologiques en chimie générale, biochimie et chimie clinique, comprenant:

deux plaques (501, 502) qui sont munies chacune sur une surface de zones réactionelles;

un bâti (bâti principal 503), maintenant les deux plaques disposées parallèlement entre elles et les empêchant de se déplacer latéralement l'une par rapport à l'autre;

des moyens (504, 505) définissant la distance la plus petite entre les deux plaques;

des moyens (506, 507) définissant la distance la plus grande entre les deux plaques;

des moyens pour changer la distance des deux plaques entre elles entre la plus grande et la plus petite valeur caracterisé par le fait que: les zones réactionelles qui sont formées de plaques hydrophiles composées de verre ou de plastique et sont entourées d'une couche hydrophobe, sont placées face à face et maintiennent les échantillons (508) des deux côtés.

2. Dispositif selon la revendication 1 pour le mélangeage simultané des échantillons (508) entre les deux plaques (501, 502) caracterisé par le fait que les moyens de changement de la distance des deux plaques entre elles sont constitués par une plaque métallique (507) attiré de façon rythmée par un électro-aimant et des ressorts de pression (509) exerçant une pression de rappel.

504

510

100 mm

Abb. 1

505

100 mm

Abb. 2

11

502

100 mm

Abb. 3

506

100 mm

Abb. 4

510

506

507

501

508

504

509

502

505

501

503

10 mm

Abb. 5: